**Europäisches Patentamt**

**European Patent Office**

**Office européen des brevets**

(19)

(11) Publication number: **0 409 550 B1**

## (12) EUROPEAN PATENT SPECIFICATION

(45) Date of publication of patent specification: **05.04.95**  (51) Int. Cl.6: **A61L 25/00**, A61K 9/70

(21) Application number: **90307785.7**

(22) Date of filing: **17.07.90**

(54) **Polymeric liquid dressing for skin.**

(30) Priority: **18.07.89 US 381556**

(43) Date of publication of application:
**23.01.91 Bulletin  91/04**

(45) Publication of the grant of the patent:
**05.04.95 Bulletin  95/14**

(84) Designated Contracting States:
**FR GB IT**

(56) References cited:
**EP-A- 0 288 336**
**WO-A-88/09185**
**GB-A- 2 188 844**
**US-A- 3 476 853**

(73) Proprietor: **ETHICON INC.**
**U.S. Route 22**
**Somerville**
**New Jersey 08876 (US)**

(72) Inventor: **Shah, Kishore R.**
**568 Cabot Hill Road**
**Bridgewater NJ 08807 (US)**
Inventor: **Ovington, Liza G.**
**1288 Stuart Road**
**Princeton, NJ 08540 (US)**
Inventor: **Doshi, Uday B.**
**97 Marvin Lane**
**Piscataway, NJ 08854 (US)**
Inventor: **Shalaby, Shalaby W.**
**328A Longview Road, Rd 2**
**Lebanon NJ 08833 (US)**

(74) Representative: **Fisher, Adrian John et al**
**CARPMAELS & RANSFORD**
**43 Bloomsbury Square**
**London WC1A 2RA (GB)**

**Description**

The invention relates to the art of liquid polymeric dressings for skin.

Background of the Invention

Liquid polymeric preparations that can be sprayed on or otherwise thinly applied to the intact or injured skin as protective coatings and/or as carriers for medicaments have been suggested for many years. They are often called "spray-on bandages" or "liquid bandages". Such preparations are disclosed, for instance, by Von Fieandt et al., U.S. Patent No. 3,269,903, who disclose dressings made of hydroxyalkyl methacrylate polymers, and Gleichenhagen et al., U.S. Patent No. 3,987,000, who disclose isobutene/acrylic copolymers as protective coatings for wounds. Other U.S. patents that disclose wound coverings made of various types of polymeric materials include the following:

| | |
|---|---|
| Wang | No. 4,793,336 |
| Gould et al. | No. 3,377,516 |
| Hofeditz et al. | No. 4,552,138 |
| Pellico | No. 4,291,025 |
| King | No. 3,419,006 |
| Korol | No. 4,563,184 |
| Gurney | No. 3,880,158 |

Commercially available liquid bandages include the following formulations:
1. Pyroxylin solution, oil of cloves, 8-hydroxyquinoline, and ethanol;
2. benzethonium chloride, dyclonine hydrochloride, and ethanol;
3. Cellulose acetate butyrate, 2-ethylhexyl diphenyl phosphate, and acetone;
4. A polymer that appears to be partially hydrolyzed vinyl chloride/vinyl acetete copolymer and a sebacic acid based polyester that is used as a polymeric plasticizer for the vinyl resin, modified maleic rosin ester, ethyl acetate, and acetone; and
5. Ethoxyethyl methacrylate polymer, tetramethyl thiuram disulfide, and ethyl acetate.

This invention relates to a polymeric formulation that can be applied to the skin in a liquid formulation which, upon evaporation of the liquid carrier in the formulation, acts as a protective coating for the intact or injured skin and can serve as a carrier for one or more medicaments. The formulation of the invention has the useful property of developing excellent adhesion to the skin, but the coating formed from the formulation has a non-sticky outer surface that does not tend to pick up dirt or to adhere to dressings. The protective film formed by the formulation of the invention is durable; it adheres to the skin at least as long as other commercially available liquid bandages, but it is more comfortable because it is compliant and more conformable to the skin. The liquid bandage of the invention also has the valuable property of having little or no tendency to sting when it is applied to the intact or injured skin.

Brief Summary of the Invention

The invention provides a polymeric formulation suitable for applying to the skin in a thin layer to form a protective coating thereon, which formulation comprises a sterile liquid comprising an ethylene/vinyl acetate copolymer which contains paraffin wax, and an organic liquid solvent for said copolymer. The organic liquid solvent comprises a mixture of a lower alkane or cycloalkane and a lower alkanol.

The Prior Art

Pospischil, in U.S. Patent No. 3,803,300, mentions that paraffin can be used as a base for an "ointment foil" for application to the skin. The ointment foil is prepared by drying an oil-in-water type emulsion to form a film with sufficient strength to be handled, and thereafter this film is applied to the skin.

WO 88/09185 discloses a spray-on film formulation which may include paraffin as a solvent.

GB-A-2188844 discloses a spray-on film formulation which may include polyethylene and polyvinyl acetate.

Detailed Description of the Invention

The polymeric formulation of the invention that is used to form a protective coating on the intact or injured skin comprises an ethylene/vinyl acetate copolymer that also contains a paraffin wax, and a liquid organic carrier. Optionally, the formulation may contain medicaments such as antibiotics and other anti-microbial agents, anti-fungal agents, corticosteroids, derivatives of retinoic acid, growth factors, non-steroidal anti-inflammatory agents, peroxides such as benzoyl peroxide, ultraviolet light absorbers, analgesics, mixtures of two or more of the above, and the like.

The polymer used in the invention is ethylene/vinyl acetate ("EVA") copolymer. The copolymer has a polymerized vinyl acetate content effective to impart adequate solvent solubility at room temperature and to improve the conformability of the copolymer to skin. For instance, the copolymer usually contains from about 30 to about 60 weight percent of polymerized vinyl acetate, the remainder being polymerized ethylene. The EVA copolymers employed in the invention usually have molecular weights such that they have inherent viscosities within the range of from about 0.3 to about 0.7 dl/gm, tested at a concentration of 0.1 gm/dl in methyl ethyl ketone at 25°C. The ethylene/vinyl acetate copolymers used in the invention are available commercially.

Paraffin wax is used in the invention to increase the modulus of elasticity of the protective film formed by the polymeric composition of the invention. The incorporation of crystalline paraffin wax in the EVA copolymer makes the protective film less rubbery and stretchy. The paraffin wax is used in an amount effective to impart to the protective coating of the invention aesthetics, the desired surface characteristics, and balance of modulus, flexibility, and conformability, without adversely affecting the adhesion of the film to the skin. Usually, the desired amount of paraffin will be found in the range of from about 2 to 25, and preferably 5 to 15, weight percent, based on the combined weight of the EVA copolymer and the wax. Routine experimentation will suffice to determine the exact proportions of wax that is desired in individual cases.

The crystalline paraffin wax used in the invention is defined as a wax having a melting point greater than about 45°C and is composed primarily of low molecular weight hydrocarbon, e. g., low molecular weight polyethylene.

The polymeric formulation of the invention contains an organic liquid vehicle that is a solvent for the EVA copolymer and preferably for the paraffin wax. A mixture of a $C_{5-8}$ alkane or cycloalkane such as hexane, cyclohexane, heptane, octane, or mixture thereof, and the like with a $C_{3-6}$ lower alkanol such as isopropyl alcohol or n-butanol has been found to be useful as the organic vehicle. Isopropyl alcohol is the preferred lower alkanol. Because the organic solvent mixture is predominantly composed of hydrocarbons, and is therefore hydrophobic, the liquid bandage of the invention has a reduced tendency to cause stinging during application to tissue. The formulation can be prepared by standard techniques, such as by mixing the paraffin wax and the EVA copolymer (in powdered or pellet form) in the alkane until the EVA copolymer is fully swollen, warming the mixture to a moderately elevated temperature below the boiling point of the alkane (e.g., 40-60°C), and then adding enough lower alkanol to cause the EVA copolymer to dissolve in the mixture. Suitable proportions of alkanol to alkane will usually be found in the range of from about 1:24 to about 1:7, by weight. The proportion of solids (EVA copolymer plus wax) to organic vehicle is not narrowly critical, and can vary from about 10 to about 35 weight percent solids, based on total weight of the formulation.

The formulation can be sterilized by conventional means such as by filtration and irradiation.

The formulation described above is suitable for applying to the unbroken or to the injured skin by brushing, spraying, or the like. By adding a propellant, the formulation may be formulated for application as an aerosol.

Example 1

Preparation of 100 gms of a liquid bandage is done by first mixing 19.8 gms of EVA copolymer (40 wt% vinyl acetate content, inherent viscosity = 0.53 dl/gm, tested at a concentration of 0.1 g/dl in methyl ethyl ketone at 25°C, and 2.2 gms of paraffin wax (mp 53-56°C) with 74.9 gms of hexane until the EVA copolymer is fully swollen. The mixture is warmed to about 50°C and 3.1 gms of isopropyl alcohol are added under continuous agitation to obtain a clear solution. The liquid polymer solution thus obtained is suitable for application to the skin by brushing or spraying. The film on the skin that is formed after the organic solvent mixture has evaporated has good adhesion to the skin and has a non-sticky surface.

3

Example 2

By procedures similar to that described above, a series of bandage formulations containing various medicaments were prepared. Table I, below, presents the proportions of the ingredients in each formulation. It is noted that a small amount of water is employed in the solvent system for several of the formulations in order to aid in the dissolution of the medicaments. When water is employed, it may be necessary to increase the proportion of lower alkanol in order to ensure a single phase formulation.

## TABLE I

### COMPOSITION (%)

| | (1) | (2) | (3) | (4) | (5) | (6) | (7) | (8) |
|---|---|---|---|---|---|---|---|---|
| EVA | 19.37 | 18.57 | 16.30 | 18.92 | 18.92 | 18.81 | 18.83 | 17.98 |
| Parrafin Wax | 2.152 | 2.063 | 1.883 | 2.103 | 2.112 | 2.090 | 2.093 | 1.997 |
| hexane | 73.28 | 70.25 | 72.45 | 71.60 | 71.93 | 71.18 | 71.26 | 68.01 |
| IPA* | 3.033 | 6.565 | 6.770 | 4.302 | 3.841 | 5.797 | 4.757 | 6.356 |
| water | 0 | 0.328 | 0.539 | 0.956 | 0.960 | 0 | 0.951 | 3.632 |
| Gramicidin (Sigma Lot# 48F-0071) | 0.005 | 0 | 0 | 0 | 0 | 0 | 0 | 0 |
| Polymixin B Sulphate (Sigma Lot# 18F-0030) | 0 | 0.026 | 0 | 0 | 0 | 0 | 0 | 0 |
| Bacitracin (Sigma Lot# 97F-0747) | 0 | 0.131 | 0.156 | 0 | 0 | 0 | 0 | 0 |
| Gentamycin Sulphate (Sigma Lot# 67F-0171) | 0 | 0 | 0 | 0.002 | 0 | 0 | 0 | 0 |
| Chlorhexidine diacetate (Sigma Lot# 57F-0542) | 0 | 0 | 0 | 0 | 0.021 | 0 | 0 | 0 |
| Tretinoin (Retin A) (Ortho Lot # 288048) | 0 | 0 | 0 | 0 | 0 | 0.020 | 0 | 0 |
| EGF* Cake (Lot# I027E97) Contains 4.96% EGF | 0 | 0 | 0 | 0 | 0 | 0 | 0.003 | 0.018 |
| total | 100 | 100 | 100 | 100 | 100 | 100 | 100 | 100 |

Notes:
* "IPA" represents isopropyl alcohol
* "EGF" represents Epidermal Growth Factor

The liquid bandage of the invention can also be applied as a protective covering for experimental wounds, including full thickness wounds, on animals. It is often necessary to create experimental wounds on animals to test the efficacy of surgical devices such as sutures and staples and therapeutic agents, including antimicrobials and growth factors. The liquid bandage can be very useful for forming a compliant protective polymeric film covering for such wounds. This covering provides least discomfort to the animal

EP 0 409 550 B1

and decreases its tendency to dislodge the protective coating. The covering of this invention presents a substantial improvement over the high Tg, less compliant, hardly conformable methacryalte polymers that are available to the animal testing community.

In a pilot study, the liquid bandage described in Example 1 was applied to full thickness wounds on a few rats. Upon drying, the applied solution formed a thin film covering the wound and the surrounding intact skin. The film bandage was well adherent to intact skin but non-adherent to the wound. The experimental animals were observed for a period of seven days. During the first three days, the protective film remained in place with only some lifting of the edges. No infection or inflammatory response was noted on or around the wound site. The film covering also remained occlusive during this time period, as evidenced by the absence of leakage of any wound exudate. Other details of the liquid bandage functionality study are noted in the surgical report given below.

LIQUID BANDAGE:

EXPERIMENTAL PROCEDURE FOR BANDAGING FULL THICKNESS WOUNDS IN THE RAT

SUMMARY

Six mature Long-Evans rats were prepared and presented for aseptic surgery. An 8 mm full thickness dermal wound was created on the dorsal surface of the cervical region. The liquid bandage was applied to the wound and surrounding skin and allowed to dry. The rats were observed daily for 5 to 7 days.

The liquid bandage was acceptable for bandaging 8 mm full thickness dermal wounds in the rat for up to 3 days. The bandage could be reapplied and resulted in decreasing area of the wound at 5 and 7 days similar to that seen with BIOCLUSIVE dressing.

CONCLUSIONS

The liquid bandage of this invention was acceptable for bandaging 8 mm full thickness dermal wounds in the rat for up to 3 days. The bandage could be reapplied and resulted in decreasing area of the wound at 5 and 7 days similar to that seen with BIOCLUSIVE dressing.

**Claims**

1. A polymeric formulation suitable for applying to the skin in a thin layer to form a protective, adherent coating thereon, which coating has a non-sticky outer surface, which formulation comprises an ethylene/vinyl acetate copolymer, paraffin wax, and an organic liquid solvent comprising a mixture of a lower alkane or cycloalkane and a lower alkanol.

2. The formulation of claim 1 wherein the organic liquid solvent comprises a mixture of hexane and isopropyl alcohol.

3. The formulation of claim 1 or claim 2 wherein said formulation further comprises a medicament.

4. The formulation of claim 1 or claim 2 wherein said formulation further comprises an agent selected from the group consisting of antimicrobial agents, analgesics, anti-inflammatory agents, growth factors, derivatives of retinoic acid, peroxides, ultraviolet light absorbers, and mixtures thereof.

5. The formulation of claim 1 or claim 2 wherein said formulation further comprises a medicament selected from the group consisting of antibiotics, anti-fungal agents, corticosteroids, tretinoin, epidermal growth factor, non-steroidal anti-inflammatory agents, benzoyl peroxide, and mixtures thereof.

6. A method for applying a protective coating to the skin which method comprises applying a sterile polymeric formulation of any of claims 1 to 3 to the skin in a thin layer and permitting the organic liquid solvent to evaporate.

7. A formulation according to any of claims 1 to 3, for use as a medical dressing.

EP 0 409 550 B1

**Patentansprüche**

1. Polymere Formulierung, die zum Auftragen auf die Haut in einer dünnen Schicht geeignet ist, um einen schützenden, haftenden Überzug darauf zu bilden, wobei der Überzug eine nicht-klebrige äußere Oberfläche aufweist, und wobei die Formulierung ein Ethylen/Vinylacetat-Copolymer, Paraffinwachs und ein organisches, flüssiges Solvens, umfassend ein Gemisch eines niederen Alkans oder Cycloalkans und eines niederen Alkanols, umfaßt.

2. Formulierung nach Anspruch 1, wobei das organische flüssige Solvens ein Gemisch aus Hexan und Isopropylalkohol umfaßt.

3. Formulierung nach Anspruch 1 oder Anspruch 2, wobei die Formulierung darüber hinaus ein Medikament umfaßt.

4. Formulierung nach Anspruch 1 oder Anspruch 2, wobei die Formulierung darüber hinaus ein Mittel umfaßt, ausgewählt aus der Gruppe bestehend aus antimikrobiellen Mitteln, Analgetika, antiinflammatorischen Mitteln, Wachstumsfaktoren, Derivate von Retinoesäure, Peroxide, ultraviolettes Licht absorbierende Mittel und Gemische davon.

5. Formulierung nach Anspruch 1 oder Anspruch 2, wobei die Formulierung darüber hinaus ein Medikament umfaßt, ausgewählt aus der Gruppe bestehend aus Antibiotika, Antipilzmittel, Corticosteroide, Tretinoin, epidermalem Wachstumsfactor, nicht-steroidalen antiinflammatorischen Mitteln, Benzoylperoxid und Gemischen davon.

6. Verfahren zum Auftragen eines schützenden Überzuges auf die Haut, wobei das Verfahren das Auftragen einer sterilen polymeren Formulierung nach einem der Ansprüche 1 bis 3 auf die Haut in einer dünnen Schicht und Verdampfenlassen des organischen flüssigen Solvens umfaßt.

7. Formulierung nach einem der Ansprüche 1 bis 3 zur Verwendung als medizinischer Verband.

**Revendications**

1. Formulation polymère appropriée pour une application sur la peau en une couche mince pour y former un revêtement adhérent et protecteur, revêtement qui possède une surface externe non collante, formulation qui comprend un copolymère éthylène/acétate de vinyle, une cire paraffinique et un solvant organique liquide comprenant un mélange d'un alcane inférieur ou d'un cycloalcane et d'un alcanol inférieur.

2. Formulation selon la revendication 1, dans laquelle le solvant organique liquide comprend un mélange d'hexane et d'alcool isopropylique.

3. Formulation selon la revendication 1 ou 2, dans laquelle ladite formulation comprend de plus un médicament.

4. Formulation selon la revendication 1 ou 2, dans laquelle ladite formulation comprend de plus un agent choisi dans le groupe formé par les agents antimicrobiens, les analgésiques, les agents anti-inflammatoires, les facteurs de croissance, les dérivés de l'acide rétinoïque, les peroxydes, les absorbants de lumière ultraviolette et leurs mélanges.

5. Formulation selon la revendication 1 ou 2, dans laquelle ladite formulation comprend de plus un médicament choisi dans le groupe formé par les antibiotiques, les agents anti-fongiques, les corticostéroïdes, la trétinoïne, le facteur de croissance épidermique, les agents anti-inflammatoires stéroidiens, le peroxyde de benzoyle et leurs mélanges.

6. Procédé pour appliquer un revêtement protecteur sur la peau, procédé qui consiste à appliquer une formulation polymère stérile selon l'une quelconque des revendications 1 à 3 sur la peau en une couche mince et à permettre au solvant liquide organique de s'évaporer.

7

**7.** Formulation selon l'une quelconque des revendications 1 à 3, pour une utilisation en tant que pansement médical.